# EUROPEAN PATENT APPLICATION

(11) **EP 3 939 648 A1**
(43) Date of publication of application: **19.01.2022**
(21) Application number: 21185608.3
(22) Date of filing: 14.07.2021
(51) Int. Cl.: A61M 25/02

(54) **FASTENER FOR MEDICAL TUBING**

(30) Priority: 14.07.2020 US 202063051475 P; 07.10.2020 US 202063088573 P; 16.06.2021 US 202117349575
(71) Applicant: Edzway LLC, Benton, AR 72015 (US)
(72) Inventor: Edds, Cameron, 72015, Benton (US); Edds, John, Lonsdale, 72087 (US)
(74) Representative: Williams Powell

(57) **Abstract**

The present invention provides a fastener to secure an intravenous tube to a patient.

## Description

### CROSS-REFERENCE TO RELATED APPLICATIONS

This application claims priority to and is a continuation-in-part of U.S. application Serial No. U.S. application 63/051,475, filed July 14, 2020, and claims priority to and is a continuation-in-part of U.S. application Serial No. U.S. application 63/088,573, filed October 7, 2020, the disclosures of which are hereby incorporated by reference in their entireties.

### STATEMENT REGARDING FEDERALLY SPONSORED RESEARCH OR DEVELOPMENT

Not applicable.

### BACKGROUND OF THE INVENTION

The invention is generally directed to a fastener for medical tubing. More specifically, the proposed invention is a clam-shell fastener to retain an intravenous tube secured to a patient to prevent the intravenous tube from becoming dislodged from the patient.

Intravenous (IV) therapy involves the administration of fluids directly into a patient's vein or artery and is commonly used to maintain or restore fluid volume and electrolyte balance, infuse medications, transfuse blood or blood components, or to administer caloric solutions that provide nourishment to patients who cannot eat or drink due to illness or impending surgical procedures. IV therapy may be administered for time periods ranging from a few hours during a discrete procedure, to more long term situations lasting for days, weeks, or more. Short term delivery methods may range from a simple syringe push, to the ubiquitous drip bag, or to a more modern pump.

IV therapy may be administered by a number of methods and is categorized by the type of vein the inserted tube, or the catheter, enters into. It is critical with all catheters, no matter what vein they're inserted into, that they stay inserted and in place. However, for some methods of IV administration, like with peripheral lines into the veins in the arms, hands, legs, and feet - the areas of the body that are most mobile for patients - the risk of inadvertent dislodgement is higher.

Current methods of securing IV tubing and catheters to patients include tape or tethers requiring tape to be applied to the skin of the patient. Examples of such inventions are taught in U.S. Pat. No. 7744572 issued to Bierman on June 29, 2010; U.S. Pat. No. 10449122 issued to Gulliver et al. on Oct. 22, 2019; and U.S. Pat. No. 7635354 issued to Navarro et al. on Dec. 22, 2009, the disclosures of which in their entirety are hereby incorporated by reference.

These prior teachings are insufficient to solve the present problem. Patients receiving long term IV therapy tend to be older or in ill health. As a result, the patient's skin is thinner and prone to tears when the IV is moved. IV therapy patients often require new catheters to be placed when the original vein becomes too weak to withstand the catheter. As a result, it is desirous to have a solution to fasten an IV tube to a patient securely without the use of tape or other adhesive substances.

Other methods of securing IV tubing and catheters to patients include removable hook and loop tethers or similar strap attachments that secure the tubing to the tether and the tether to the patient. Examples of such inventions are taught in U.S. Pat. No. 5,664,581 issued to Ashley on Sept. 9, 1997, U.S. Pat. No. 4846807 issued to Safadago on July 11, 1989; U.S. Pat. No. 9518667 issued to Ramos et al. on Dec. 13, 2016; U.S. Pat. No. 5342317 issued to Claywell on Aug. 30, 1994; U.S. Pat. No. 5413562 issued to Swauger on May 9, 1995; and U.S. Pub. No. 20200023120 published for Fischer on January 23, 2020, the disclosures of which in their entirety are hereby incorporated by reference.

These prior teachings are insufficient to solve the present problem. Patients receiving IV therapy are often still mobile, and, as a result, they may move the peripheral limb that the IV tubing and catheter are affixed. The tethers taught by the prior art fail to teach a fastener to the patient that restricts the movement of the IV tubing between the catheter end and the location on the patient's body, so that when the patient eventually moves her limb, the IV catheter remains in place despite movement of the IV tubing beyond the fastener position on the patient's limb.

Therefore, there is a need in the art for a fastener which can be secured to a patient's body that restricts the movement of the IV tubing between the catheter and the fastener location.

### STATEMENT OF INVENTION

According to an aspect of the present invention, there is provided a fastener as claimed in claim 1.

An embodiment of the present invention is directed to a clam-shell fastener to retain an intravenous tube secured to a patient to prevent the intravenous tube from becoming dislodged from the patient.

The present invention seeks to provide a device that secures an intravenous tube to a patient to prevent catheter dislodgement.

The present invention further seeks to provide a device that securely holds an intravenous tube to a patient without the use of tape.

The present invention further seeks to provide a removable device that can be placed on the extremities of a patient to secure an intravenous tube.

In one embodiment, a fastener for securing medical tubing to a patient comprises a lid extending a length along a longitudinal axis, the lid having an arcuate exterior and an interior surface, a base in a hingeable relationship with the lid, the base extending a length along a longitudinal axis from a first side to an opposing second side and having an interior surface; an arcuate engaging channel extending a length along the longitudinal axis of the base from the first side of the base to the opposing second side of the base, the arcuate engaging channel having a first channel side and an opposing second channel side each extending the length of the arcuate engaging channel, the arcuate engaging channel further comprising a series of engaging projections configured to grip the medical tubing, each engaging projection of the series of engaging projections extending from the engaging channel from the first channel side of the arcuate engaging channel to the second channel side and distributed along the length of the arcuate engaging channel; a series of supporting ribs extending from the interior surface of the lid along the longitudinal axis of the lid; an engaging strip extending from the interior surface of the lid along the entire longitudinal length of the lid, the engaging strip having a multitude of teeth distributed along the engaging strip.

In another embodiment, a fastener for securing medical tubing to a patient comprises a lid extending a length along a longitudinal axis, the lid having an interior surface, a base in a hingeable relationship with the lid, the base extending a length along a longitudinal axis from a first side to an opposing second side and having an interior surface; an arcuate engaging channel extending a length along the longitudinal axis of the base from the first side of the base to the opposing second side of the base, the arcuate engaging channel having a first channel side and an opposing second channel side each extending the length of the arcuate engaging channel, the arcuate engaging channel further comprising a series of engaging projections configured to grip the medical tubing, each engaging projection of the series of engaging projections extending from the engaging channel from the first channel side of the arcuate engaging channel to the second channel side and distributed along the length of the arcuate engaging channel; an engaging strip extending from the interior surface of the lid along the entire longitudinal length of the lid; and a closure configured to secure the lid to the base.

In another embodiment, a fastener for securing medical tubing to a patient comprises a housing having a first piece having an interior surface and a second piece having an opposing interior surface, the housing extending a length along a longitudinal axis; an engaging channel extending a length along the longitudinal axis of the housing on the interior surface of the second piece from the first side of the second piece to the opposing second side of the second piece, the engaging channel having a first channel side and an opposing second channel side each extending the length of the engaging channel, the engaging channel further comprising a series of engaging projections configured to grip the medical tubing, each engaging projection of the series of engaging projections extending from the engaging channel from the first channel side of the engaging channel to the second channel side and distributed along the length of the engaging channel; and an engaging strip extending from the interior surface of the first piece along a portion of the longitudinal length of the housing.

### BRIEF DESCRIPTION OF THE DRAWINGS

Fig. 1 is an environmental view of the fastener for medical tubing of the present invention, with the fastener open;
Fig. 2 is an environmental view of the fastener for medical tubing of the present invention, with the fastener closed;
Fig. 3 is a top perspective view of the fastener for medical tubing of the present invention;
Fig. 4 is a bottom perspective view of the fastener for medical tubing of the present invention;
Fig. 5 is a left side elevational view of the fastener for medical tubing of the present invention;
Fig. 6 is a right side elevational view of the fastener for medical tubing of the present invention;
Fig. 7 is a top plan view of the fastener for medical tubing of the present invention;
Fig. 8 is a bottom plan view of the fastener for medical tubing of the present invention;
Fig. 9 is a rear elevational view of the fastener for medical tubing of the present invention;
Fig. 10 is a front plan view of the fastener for medical tubing of the present invention;
Fig. 11 is a top perspective view of a second embodiment of the fastener for medical tubing of the present invention;
Fig. 12 is a left side elevational view of the second embodiment of the fastener for medical tubing of the present invention;
Fig. 13 is a front plan view of the second embodiment of the fastener for medical tubing of the present invention;
Fig. 14 is a top plan view of the second embodiment of the fastener for medical tubing of the present invention;
Fig. 15 is a top perspective view of a third embodiment of the fastener for medical tubing of the present invention;
Fig. 16 is a bottom perspective view of a third embodiment of the fastener for medical tubing of the present invention;
Fig. 17 is a right side elevational view of a third embodiment of the fastener for medical tubing of the present invention;
Fig. 18 is a left side elevational view of a third embodiment of the fastener for medical tubing of the present invention;
Fig. 19 is a top plan view of a third embodiment of the fastener for medical tubing of the present invention;
Fig. 20 is a top perspective view of a fourth embodiment of the fastener for medical tubing of the present invention;
Fig. 21 is a bottom perspective view of a fourth embodiment of the fastener for medical tubing of the present invention;
Fig. 22 is a right side elevational view of a fourth embodiment of the fastener for medical tubing of the present invention;
Fig. 23 is a left side elevational view of a fourth embodiment of the fastener for medical tubing of the present invention;
Fig. 24 is a top plan view of a fourth embodiment of the fastener for medical tubing of the present invention;
Fig. 25 is a top perspective view of a fifth embodiment of the fastener for medical tubing of the present invention;
Fig. 26 is a left side elevational view of a fifth embodiment of the fastener for medical tubing of the present invention;
Fig. 27 is a right side elevational view of a fifth embodiment of the fastener for medical tubing of the present invention;
Fig. 28 is a top plan view of a fifth embodiment of the fastener for medical tubing of the present invention;
Fig. 29 is a top perspective view of the engaging portion of a sixth embodiment of the fastener for medical tubing of the present invention;
Fig. 30 is a top perspective view of the engaging portion of the sixth embodiment of the fastener for medical tubing of the present invention;
Fig. 31 is a front elevational view of the engaging portion of the sixth embodiment of the fastener for medical tubing of the present invention;
Fig. 32 is a top plan view of the engaging portion of the sixth embodiment of the fastener for medical tubing of the present invention;
Fig. 33 is a rear elevational view of the engaging portion of the sixth embodiment of the fastener for medical tubing of the present invention;
Fig. 34 is a top perspective view of the engaging portion of a seventh embodiment of the fastener for medical tubing of the present invention;
Fig. 35 is a top perspective view of the engaging portion of the seventh embodiment of the fastener for medical tubing of the present invention;
Fig. 36 is a top plan view of the engaging portion of the seventh embodiment of the fastener for medical tubing of the present invention;
Fig. 37 is a rear elevational view of the engaging portion of the seventh embodiment of the fastener for medical tubing of the present invention;
Fig. 38 is a top perspective view of the engaging portion of an eighth embodiment of the fastener for medical tubing of the present invention;
Fig. 39 is a rear elevational view of the engaging portion of the eighth embodiment of the fastener for medical tubing of the present invention;
Fig. 40 is a top plan view of the engaging portion of the eighth embodiment of the fastener for medical tubing of the present invention;
Fig. 41 is a top perspective view of the engaging portion of a ninth embodiment of the fastener for medical tubing of the present invention;
Fig. 42 is a rear elevational view of the engaging portion of the ninth embodiment of the fastener for medical tubing of the present invention;
Fig. 43 is a top plan view of the engaging portion of the ninth embodiment of the fastener for medical tubing of the present invention;
Fig. 44 is a top perspective view of a tenth embodiment of the fastener for medical tubing of the present invention;
Fig. 45 is a bottom perspective view of the tenth embodiment of the fastener for medical tubing of the present invention;
Fig. 46 is a left side elevational view of the tenth embodiment of the fastener for medical tubing of the present invention;
Fig. 47 is a right side elevational view of the tenth embodiment of the fastener for medical tubing of the present invention;
Fig. 48 is a top perspective view of the engaging portion of an eleventh embodiment of the fastener for medical tubing of the present invention;
Fig. 49 is a top perspective view of the engaging portion of an eleventh embodiment of the fastener for medical tubing of the present invention;
Fig. 50 is a right side elevational view of the engaging portion of an eleventh embodiment of the fastener for medical tubing of the present invention;
Fig. 51 is a left side view of the engaging portion of an eleventh embodiment of the fastener for medical tubing of the present invention;
Fig. 52 is a top perspective view of an IV extension bracelet for a twelfth embodiment of the fastener for medical tubing of the present invention;
Fig. 53 is a bottom perspective view of an IV extension bracelet for the twelfth embodiment of the fastener for medical tubing of the present invention;
Fig. 54 is a left side elevational view of an IV extension bracelet for the twelfth embodiment of the fastener for medical tubing of the present invention;
Fig. 55 is a right side elevational view of an IV extension bracelet for the twelfth embodiment of the fastener for medical tubing of the present invention;
Fig. 56 is a front plan view of an IV extension bracelet for the twelfth embodiment of the fastener for medical tubing of the present invention;
Fig. 57 is a rear plan view of an IV extension bracelet for the twelfth embodiment of the fastener for medical tubing of the present invention;
Fig. 58 is a top perspective view of an IV extension bracelet with a breakaway device for a thirteenth embodiment of the fastener for medical tubing of the present invention;
Fig. 59 is a top perspective view of a double luer bracelet for a fourteenth embodiment of the fastener for medical tubing of the present invention;
Fig. 60 is a bottom perspective view of a double luer bracelet for the fourteenth embodiment of the fastener for medical tubing of the present invention;
Fig. 61 is a front plan view of a double luer bracelet for the fourteenth embodiment of the fastener for medical tubing of the present invention, the opposing side being a mirror image thereof;
Fig. 62 is a side elevational view of the double luer bracelet for the fourteenth embodiment of the fastener for medical tubing of the present invention, the opposing side being a mirror image thereof;
Fig. 63 is a top plan view of the double luer bracelet for the fourteenth embodiment of the fastener for medical tubing of the present invention;
Fig. 64 is a top perspective view of the flexible clip bracelet for a fifteenth embodiment of the fastener for medical tubing of the present invention;
Fig. 65 is a bottom perspective view of the flexible clip bracelet for the fifteenth embodiment of the fastener for medical tubing of the present invention;
Fig. 66 is a top plan view of the flexible clip bracelet for the fifteenth embodiment of the fastener for medical tubing of the present invention;
Fig. 67 is a side elevational view of the flexible clip bracelet for the fifteenth embodiment of the fastener for medical tubing of the present invention, the opposing side being a mirror image;
Fig. 68 is a front elevational view of the flexible clip bracelet for the fifteenth embodiment of the fastener for medical tubing of the present invention; and
Fig. 69 is a rear elevational view of the flexible clip bracelet for the fifteenth embodiment of the fastener for medical tubing of the present invention.

### DETAILED DESCRIPTION OF THE INVENTION

Generally speaking, the present invention is directed to a fastener for medical tubing. More specifically, the proposed invention is a clam-shell fastener to retain an intravenous tube secured to a patient to prevent the intravenous tube from becoming dislodged from the patient.

The term fastener as used herein, refers to any object, mechanism, means or technique capable of securing an item to another item.

As used herein, the term "hook and loop type fasteners" refers to fastening means comprising a "hook" component (hereinafter also referred to as an "engaging component") and a complementary loop component (hereinafter also referred to as a "landing component"). The term "hook" is used to designate a material having engaging elements. Thus, the hook fastening material may also be referred to as a male fastener. It should also be understood that the use of the term "hook" should be nonlimiting in the sense that the engaging elements may comprise any shapes as are known in the art so long as they are adapted to engage a complementary landing component.

The term patient encompasses, but is not limited to, a recipient of health care services.

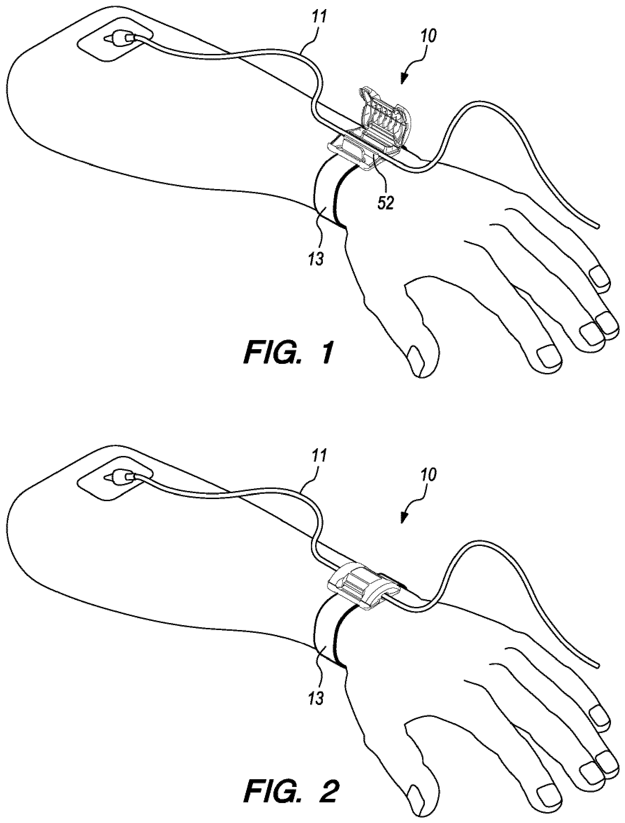

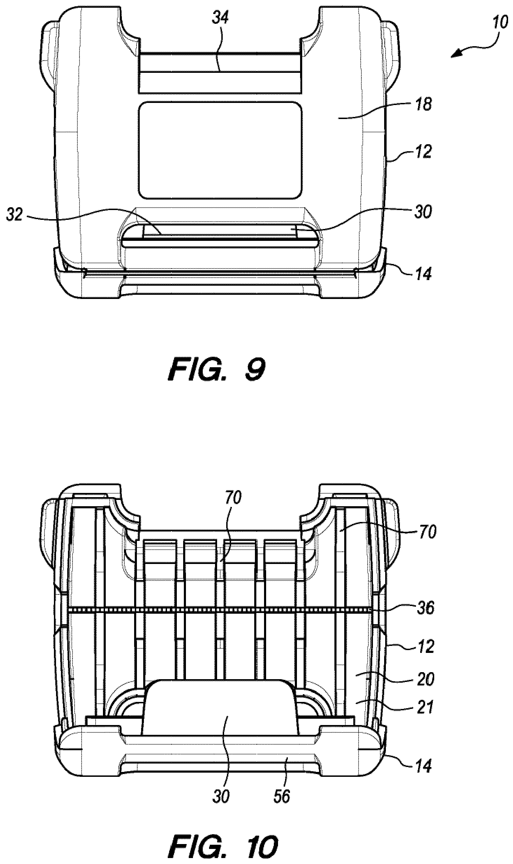

Fig. 1 - 10 show the first embodiment of the fastener for medical tubing. Fig. 1 and Fig. 2 both show the fastener 10 for medical tubing secured to an arm of a patient with a hook and loop strip or other attachment means 13 and with medical tubing 11 running from a catheter in the patient's arm through the fastener 10. As shown in Fig. 1, the medical tubing 11 is secured in the engaging channel 52 and as shown in Fig. 2, the fastener 10 comprises a clam-shell housing structure having a first piece or lid piece 12 in a hingeable relationship with a second piece or base piece 14 to enclose within the fastener 10 medical tubing or intravenous tubing to restrict the movement of the tubing.

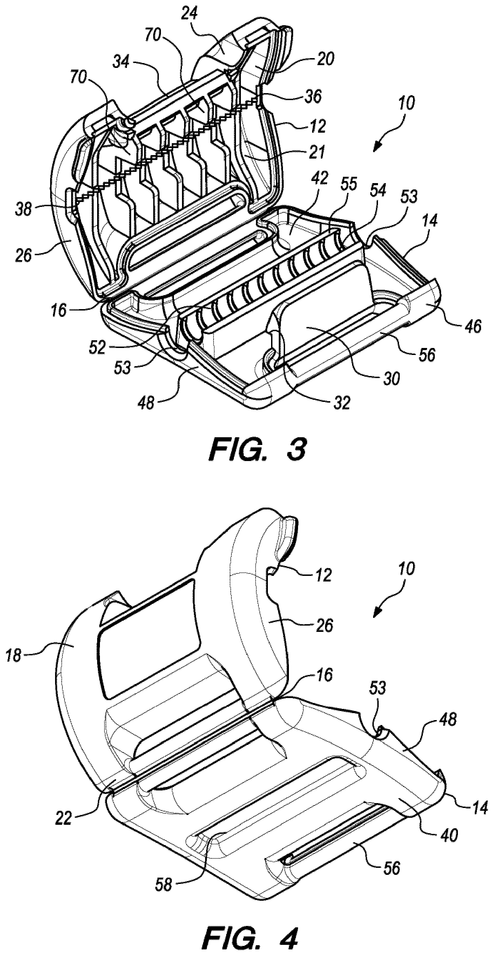

Fig. 3 is a top perspective view of the fastener for medical tubing of the present invention. The hingeable relationship between the first piece 12 and the second piece 14 is created by a hinge 16 secured to the first piece 12 and the second piece 14. The fastener 10 can be composed of different materials suitable for fasteners, including metal and plastic. The first piece and second piece will be discussed more fully below and their respective components.

As shown in the first embodiment shown in Figures 1 - 10, the first piece 12 has an arcuate exterior face 18, an interior compartment 20 extending along the longitudinal axis of the first piece 12 and that is enclosed within the fastener 10 when the fastener is closed, a rear side 22, and front side 24, and a left and right side 26, 28. A closure 30 having a locking projection 32 is secured to the front side 24 of the first piece 12 and configured to connect and secure to a corresponding inlet 34 on the second piece 14 to secure the first piece to the second piece along the front face 24 of the fastener 10.

A series of supporting ribs 70 extend outward from the interior surface 21 along the longitudinal axis of the first piece 12 from the left side 26 to the right side 28 on the interior surface 21 to support the arcuate exterior face 18 and prevent compression on the exterior of the lid 12 when engaged with the base 14. The interior compartment 20 includes an engaging strip 36 located in the center of the surface 21 and running parallel to the longitudinal axis of the fastener from the left 26 to the right side 28 of the fastener 10. The engaging strip 36 is composed of a multitude of small teeth 38 configured to grip but not puncture an intravenous tube placed within the fastener. The multitude of teeth 28 are distributed along the engaging strip 36, the distance between two consecutive teeth being constant throughout the distribution.

The second piece 14 has an exterior face 40, an interior surface 42 that is enclosed within the fastener 10 when the fastener is closed and that extends along the longitudinal axis of the base 14, a rear side 44, and front side 46, and a left and right side 48, 50. The closure flap 30 locking projection 32 of the first piece 12 is configured to connect and secure to the corresponding inlet 34 on the front side 46 of the second piece 14 to secure the first piece to the second piece along the front face 46 of the fastener 10.

The interior 42 of the second piece 14 includes an engaging channel 52 located in the center of the face and running parallel to the longitudinal axis of the fastener from the left 48 to the right side 50 of the fastener 10 piece 14. Each side 48, 50 of the piece have a corresponding channel aperture 53 to allow for the retention of the intravenous tube. The engaging channel 52 is configured to retain within the channel 52 an intravenous tube placed within the fastener. The channel 52 has two opposing channel sides 55, 57 which extend the length of the channel 52. The channel 52 includes a series of engaging projections 54 perpendicular to the longitudinal axis of the fastener 10 configured to grip but not puncture or clamp an intravenous tube retained within the channel to hold the tubing from moving along the channel 52. The engaging projections 54 are distributed along the engaging channel, the distance between two consecutive projections being constant throughout the distribution.

As shown, when the first piece is hingeably closed upon the second piece 14, the engaging strip 36 is positioned parallel and above the engaging channel 52. In this manner, an intravenous tube placed within the fastener 10 is engaged along the first piece by the engaging strip 36 and the multitude of engaging teeth 38 and retained within the engaging channel 52 and held in place by the engaging projections 54.

Fig. 4 is a bottom perspective view of the fastener for medical tubing of the present invention. As shown the exterior face 40 of the second piece 14 contains attachment ports 56, 58 for attachment means 13 for the fastener 10 to secure the fastener to a human limb. It is envisioned that a hook and loop strip or other binding strips 13 may be secured to the attachment ports 56, 58.

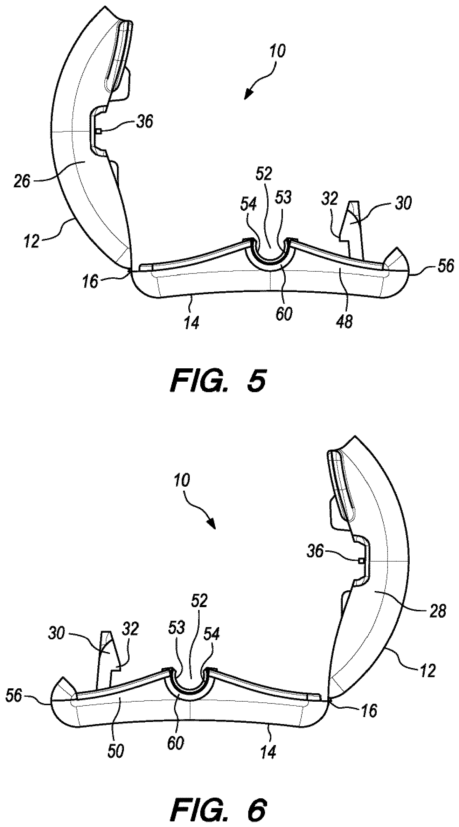

Fig. 5 and Fig. 6 detail the left and right side of the fastener 10 for medical tubing. As shown, the engaging strip of the first piece 12 extends to both the left and right face of the fastener 10 and positioned parallel and above the engaging channel 52. From the left and right side, the arcuate cross-section of the engaging channel 52 is visible. The engaging channel 52 extends from the left to the right face of the fastener 10 and has a channel face 60. The engaging projections 54, as viewed from the left and right side of the fastener 10, extend upward from the channel face 60. It is envisioned that other methods of engaging the IV tubing within the engaging channel 52 could be used in addition or in substitution for the engaging projections 54. These alternatives include small-scale texturing of the engaging channel 54 or embedding of non-adhesive, high-friction materials within the engaging channel 54.

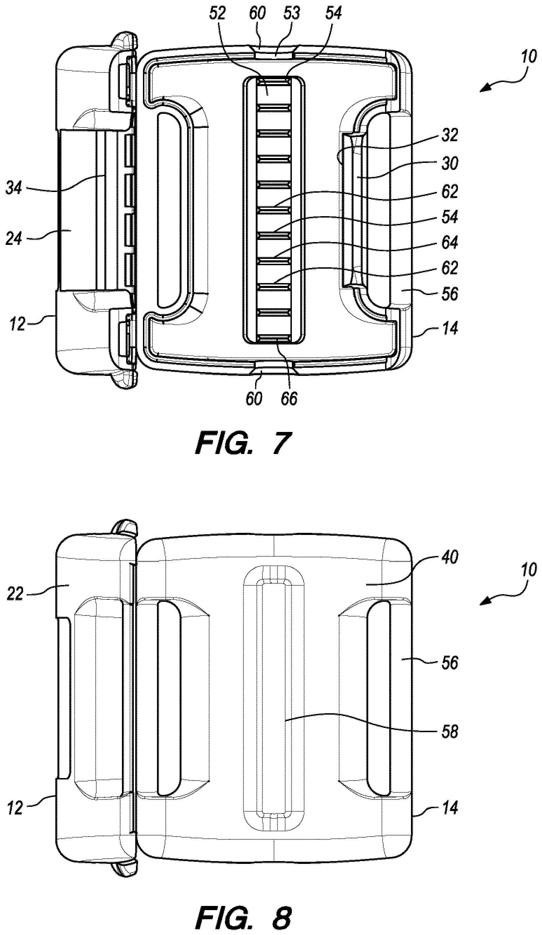

Fig. 7 is a top plan view of the fastener for medical tubing. As shown from the top, the thickness of the engaging projections 54 are visible within the channel 52. Each engaging projection 54 has a circular cross-section 66 and within the channel, has a top edge 62 and arcuate sides 64 extending on both sides of the projection from the top edge 62 to the channel face 60.

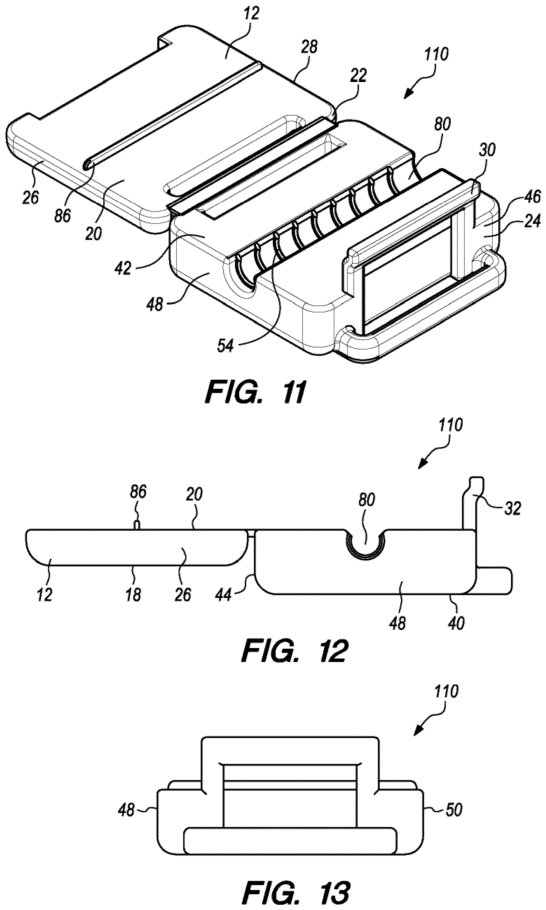

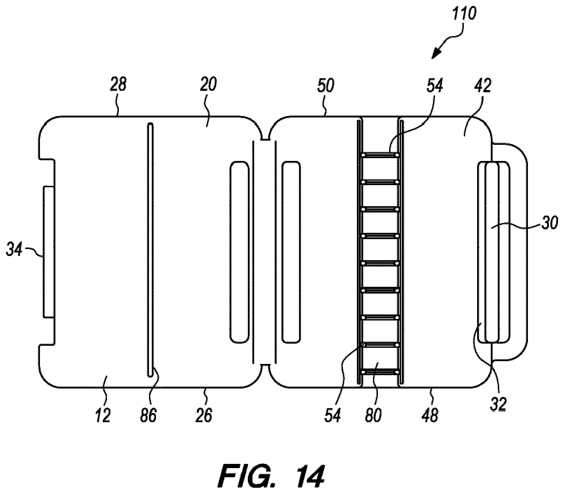

A second embodiment is presented in Figs. 11 - 14, the first piece 12 is a substantially planar piece having an exterior face 18, an interior face 20 that is enclosed within the fastener 110 when the fastener is closed, a rear side 22, and front side, and a left and right side 26, 28. A closure flap 30 having a locking projection 32 is secured to the front side 24 of the second piece 14 and configured to connect and secure to a corresponding inlet 34 on the first piece 12 to secure the first piece to the second piece along the front face 24 of the fastener 110. The second piece 14 is a substantially parallelepiped shaped piece having an exterior face 40, an interior face 42 that is enclosed within the fastener 110 when the fastener is closed, a rear side 44, and front side 46, and a left and right side 48, 50.

The interior face 42 of the second piece 14 includes an engaging channel 80 located in the center of the face and running parallel to the longitudinal axis of the fastener from the left 48 to the right side 50 of the fastener 110 piece 14. The engaging channel 80 is configured to retain within the channel 80 an intravenous tube placed within the fastener. The channel 80 includes a series of engaging projections 54 perpendicular to the longitudinal axis of the fastener 10 configured to grip but not puncture or clamp an intravenous tube retained within the channel to hold the tubing from moving along the channel 80. The engaging projections 54 are distributed along the engaging channel, the distance between two consecutive projections being constant throughout the distribution.

The interior compartment 20 of the first piece 14 includes an engaging strip 86 located in the center of the face and running parallel to the longitudinal axis of the fastener from the left 26 to the right side 28 of the fastener 110. As shown, when the first piece is hingeably closed upon the second piece 14, the engaging strip 86 is positioned parallel and above the engaging channel 80. In this manner, an intravenous tube placed within the fastener 110 is engaged and biased within the engaging channel 80. The engaging strip 86 is smooth and may be coated in a high friction polymer or adhesive materials.

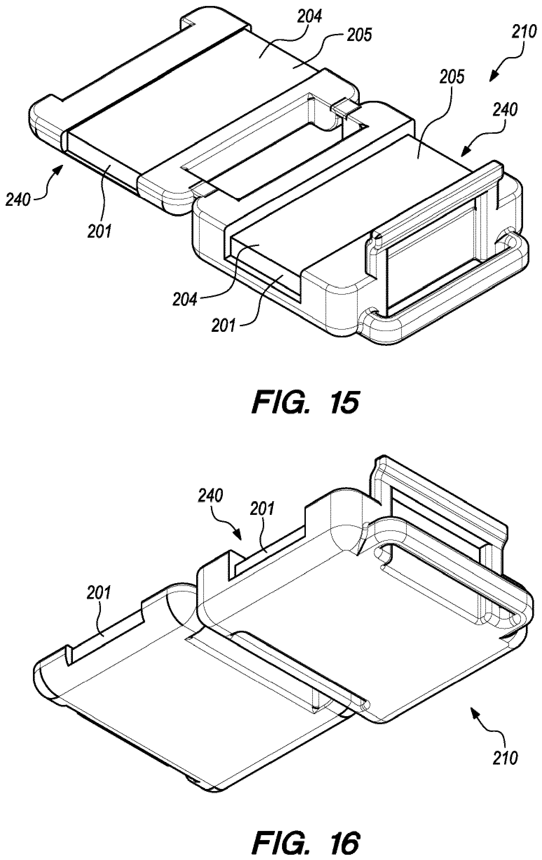

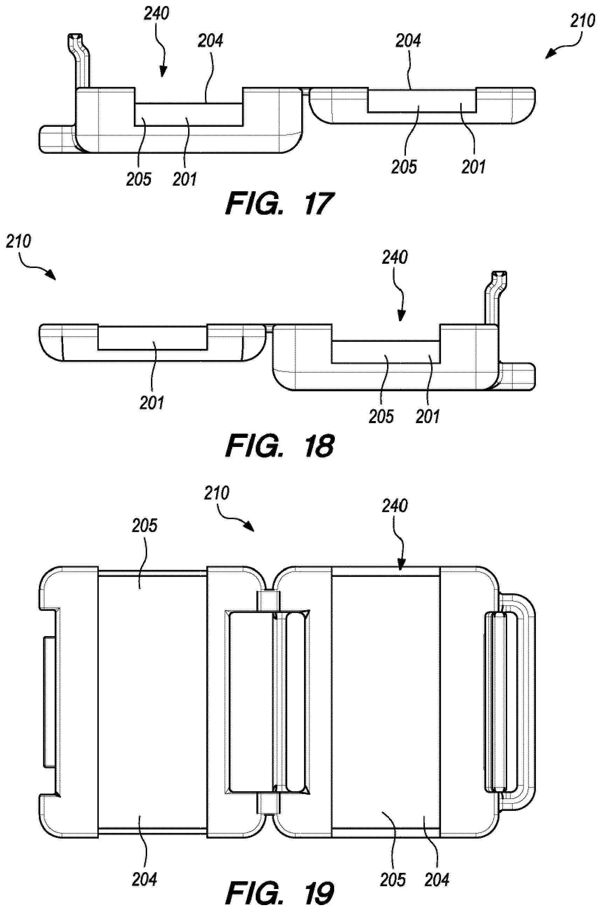

Fig. 15 - 19 show the third embodiment of the fastener 210 for medical tubing. Fig. 15 is a top perspective view showing the fastener 210 for medical tubing having a layer of gel 201 in the engaging channels. In this embodiment, the gel 201 is an adhesive or non-adhesive polyurethane or silicone that is deformable. Alternatively, the gel 201 may be replaced by memory foam, sponge, or any other similar soft, reversibly compressible materials. Optionally, overlaying the gel layer 201 is a thin layer 204 of a high friction polymer, such as non-adhesive silicon rubber. The combination of the high friction polymer layer 204 and the gel layer 201 forms a deformable cushion that allows the channel 240 to mold itself to different tubing diameters. Alternatively, the adhesive gel layer 201 may be employed without the overlying polymer layer 204. This allows the gel layer 201 to adhere to IV tubing placed into the channel 240. As with the first and second embodiment, the fastener 210 includes a first piece 12 that is a substantially planar piece having an exterior face 18, an interior face 20 that is enclosed within the fastener 210 when the fastener is closed, a rear side 22, and front side 24, and a left and right side 26, 28. A closure flap 30 having a locking projection 32 is secured to the front side 24 of the first piece 12 and configured to connect and secure to a corresponding inlet 34 on the second piece 14 to secure the first piece to the second piece along the front face 24 of the fastener 210.

The interior face 20 of the first piece 12 includes an engaging channel 240 located in the center of the face and running parallel to the longitudinal axis of the fastener from the left 26 to the right side 28 of the fastener 110. The engaging channel 240 is of sufficient width to retain an intravenous tube placed within the fastener and has an engaging face 84 that has the gel layer 201 and high friction layer 204 cushion within the channel 240. The cushion 205 is distributed along the engaging channel 240.

The second piece 14 is a substantially planar piece having an exterior face 40, an interior face 42 that is enclosed within the fastener 210 when the fastener is closed, a rear side 44, and front side 46, and a left and right side 48, 50. The closure flap 30 locking projection 32 of the first piece 12 is configured to connect and secure to the corresponding inlet 34 on the front side 46 of the second piece 14 to secure the first piece to the second piece along the front face 46 of the fastener 210.

The interior face 42 of the second piece 14 includes an engaging channel 240 located in the center of the face and running parallel to the longitudinal axis of the fastener from the left 48 to the right side 50 of the fastener 210 piece 14. The engaging channel 240 is configured to retain within the channel 52 an intravenous tube placed within the fastener. The engaging channel 240 is of sufficient width to retain an intravenous tube placed within the fastener and has an engaging face 78 that has a gel layer 201 and high friction layer 204 cushion 205. The cushion 205 is distributed along the engaging channel.

As shown, when the first piece is hingeably closed upon the second piece 14, the first engaging channel 240 is positioned parallel and above the second engaging channel 240. In this manner, an intravenous tube placed within the fastener 210 is engaged within the first and second engaging channels 240 and held in place by the gel layer 201 and high friction layer 204 cushion 240 extending along the length of the channels 240.

It is envisioned that the hingeable relationship between the first and second pieces for all embodiments discussed herein could be substituted for other locking arrangements, such as pieces having a snap configuration, a sliding configuration, a pivot configuration, pinned configuration, a fastened configuration using a screw, buckle, latch or bolt, a tied configuration, or any other resilient means of fastening the first and second pieces.

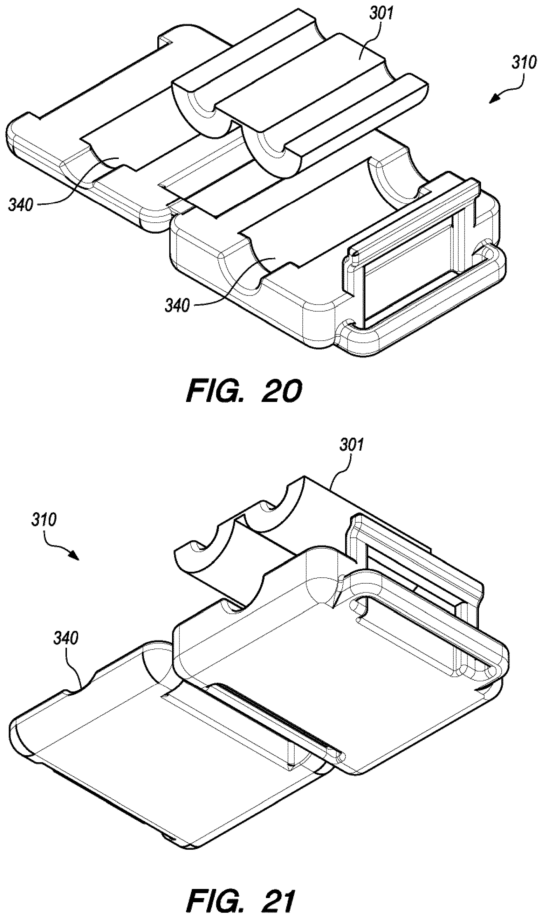

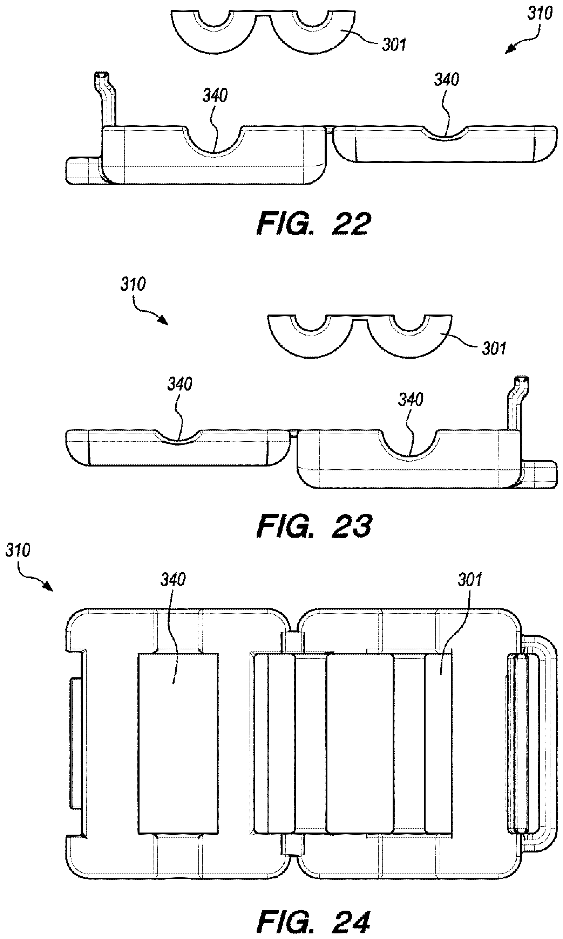

Fig. 20 - 24 show the fourth embodiment of the fastener 310 for medical tubing. Fig. 20 is a top perspective view showing the fastener 310 for medical tubing having an IV tube-gripping channel 301 in the engaging channels. In this embodiment, the IV tube-gripping channel 301 is composed of an adhesive inside a plastic, rubber, etc. clamshell tube that can be adhered outside of the fastener 310 to the IV prior to placement in the fastener 301. The IV tube-gripping channel 301 contains a deformable material, such as a gel adhesive, that allows the channel 301 to mold itself to different tubing diameters. The channel 301 is then placed within the fastener in two hemi-cylindrical cavities 340 in the fastener halves. As with the other embodiments, the fastener 310 includes a first piece 12 that is a substantially planar piece having an exterior face 18, an interior face 20 that is enclosed within the fastener 310 when the fastener is closed, a rear side 22, and front side 24, and a left and right side 26, 28. A closure flap 30 having a locking projection 32 is secured to the front side 24 of the first piece 12 and configured to connect and secure to a corresponding inlet 34 on the second piece 14 to secure the first piece to the second piece along the front face 24 of the fastener 310.

The interior face 20 of the first piece 12 includes an engaging channel 340 located in the center of the face and running parallel to the longitudinal axis of the fastener from the left 26 to the right side 28 of the fastener 110. The engaging channel 340 is of sufficient width to retain the IV tube-gripping channel 301 placed within the fastener 310.

The second piece 14 is a substantially planar piece having an exterior face 40, an interior face 42 that is enclosed within the fastener 310 when the fastener is closed, a rear side 44, and front side 46, and a left and right side 48, 50. The closure flap 30 locking projection 32 of the first piece 12 is configured to connect and secure to the corresponding inlet 34 on the front side 46 of the second piece 14 to secure the first piece to the second piece along the front face 46 of the fastener 310.

The interior face 42 of the second piece 14 includes an engaging channel 340 located in the center of the face and running parallel to the longitudinal axis of the fastener from the left 48 to the right side 50 of the fastener 310 piece 14. The engaging channel 240 is configured to retain within the channel 52 the IV tube-gripping channel 301.

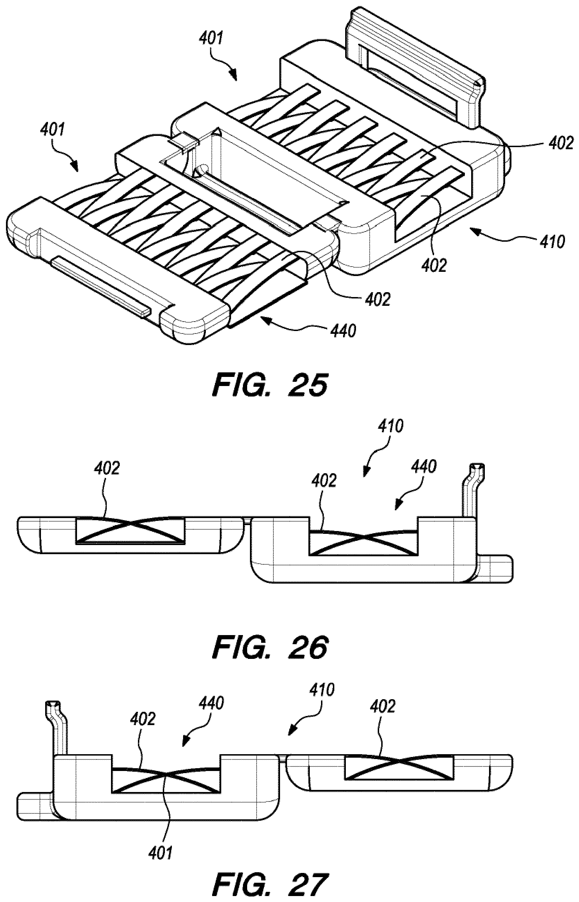

Fig. 25 - 28 show the fifth embodiment of the fastener 410 for medical tubing. Fig. 25 is a top perspective view showing the fastener 410 for medical tubing having a series 401 of arch spring clips 402 in the engaging channels 440. In this embodiment, the IV tube in the engaging channels 440 is gripped by the series of clips 402, which may include a layer of high friction polymer on the side contacting the IV tube or a layer of teeth or texture to contact the tubing. As with the other embodiments, the fastener 410 includes a first piece 12 that is a substantially planar piece having an exterior face 18, an interior face 20 that is enclosed within the fastener 410 when the fastener is closed, a rear side 22, and front side 24, and a left and right side 26, 28. A closure flap 30 having a locking projection 32 is secured to the front side 24 of the first piece 12 and configured to connect and secure to a corresponding inlet 34 on the second piece 14 to secure the first piece to the second piece along the front face 24 of the fastener 410.

The interior face 20 of the first piece 12 includes an engaging channel 440 located in the center of the face and running parallel to the longitudinal axis of the fastener from the left 26 to the right side 28 of the fastener 410. The engaging channel 440 is of sufficient width to retain the series 401 of arch spring clips 402 placed within the fastener 410.

The second piece 14 is a substantially planar piece having an exterior face 40, an interior face 42 that is enclosed within the fastener 410 when the fastener is closed, a rear side 44, and front side 46, and a left and right side 48, 50. The closure flap 30 locking projection 32 of the first piece 12 is configured to connect and secure to the corresponding inlet 34 on the front side 46 of the second piece 14 to secure the first piece to the second piece along the front face 46 of the fastener 410.

The interior face 42 of the second piece 14 includes an engaging channel 440 located in the center of the face and running parallel to the longitudinal axis of the fastener from the left 48 to the right side 50 of the fastener 410 piece 14. The engaging channel 240 is configured to retain within the channel 52 the series 401 of arch spring clips 402.

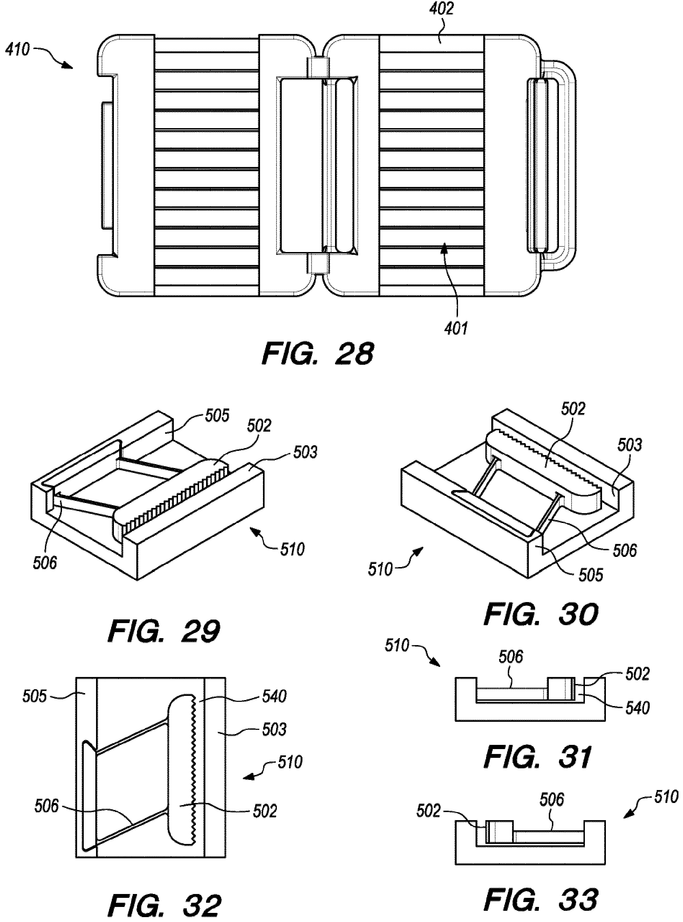

Fig. 29 - 33 show the mechanism for the sixth embodiment of the fastener 510 for medical tubing without the exterior fastener. In this embodiment, the cam gripping mechanism 510 is placed within the fastener in the two cavities in the fastener halves. As with the other embodiments, the fastener includes a first piece that is a substantially planar piece having an exterior face, an interior face that is enclosed within the fastener when the fastener is closed, a rear side, and front side, and a left and right side.

The interior faces of the first piece and second pieces include an engaging channel 540 of sufficient width to retain the cam mechanism 510 within the fastener. The cam mechanism, as shown in Figures 29 - 32, has a series of gripping teeth 502 proximate the side of the engaging channel 503 sufficient to retain an IV tubing between the gripping teeth 502 and the wall 503 of the engaging channel 540. The series of gripping teeth 502 are biased against the engaging channel wall 503 by at least one arm 506 extending from the opposing engaging channel wall 505 to the back of the series of gripping teeth 502.

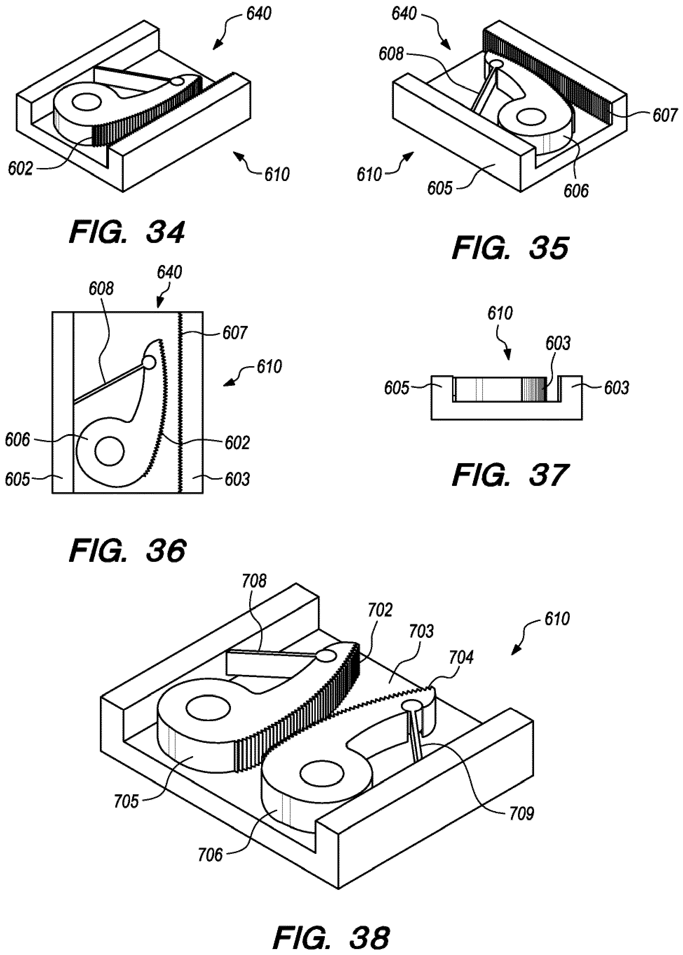

Fig. 34 - 37 show the mechanism for the seventh embodiment of the fastener 610 for medical tubing. In this embodiment, the cam gripping mechanism 610 is placed within the fastener in the two cavities in the fastener halves. As with the other embodiments, the fastener includes a first piece that is a substantially planar piece having an exterior face, an interior face that is enclosed within the fastener when the fastener is closed, a rear side, and front side, and a left and right side.

The interior faces of the first piece and second pieces include an engaging channel 640 of sufficient width to retain the cam mechanism 610 within the fastener. The cam mechanism, as shown in Figures 34 - 36, has a first series of gripping teeth 602 proximate the side of the engaging channel 603 and a second series of gripping teeth 607 along the side of the engaging channel wall 603 sufficient to retain an IV tubing between the gripping teeth 602 and the wall 603 of the engaging channel 640. The series of gripping teeth 602 are biased against the engaging channel wall 603 by at least one arm 608 extending from the opposing engaging channel wall 605 to the back of the series of gripping teeth 602 and a rotary cam piece 606 biased against the opposing engaging channel wall 605.

Fig. 38 - 40 show the mechanism for the eighth embodiment of the fastener 710 for medical tubing. In this embodiment, the cam gripping mechanism 710 is placed within the fastener in the two cavities in the fastener halves. As with the other embodiments, the fastener includes a first piece that is a substantially planar piece having an exterior face, an interior face that is enclosed within the fastener when the fastener is closed, a rear side, and front side, and a left and right side.

The interior faces of the first piece and second pieces include an engaging channel 740 of sufficient width to retain the cam mechanism 710 within the fastener. The cam mechanism, as shown in Figures 38 - 40, has a first series of gripping teeth 702 on a first cam 705 and proximate the side of the engaging channel 703 and a second series of gripping teeth 704 on a second opposing cam 706 along the side of the engaging channel 703 sufficient to retain an IV tubing between the first cam gripping teeth 702 and the second cam gripping teeth 703. The series of gripping teeth 702, 704 each are biased towards the engaging channel 703 by at least one arm 708, 709 extending from the opposing engaging channel wall and a rotary cam piece 705, 706 biased against the opposing engaging channel wall.

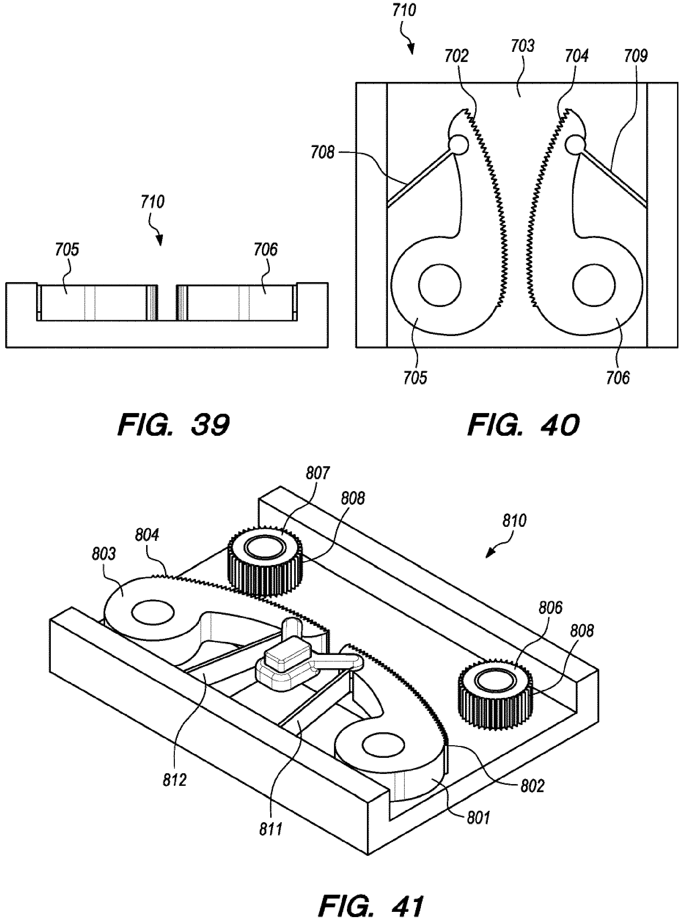

Fig. 41 - 43 show the mechanism for the ninth embodiment of the fastener 810 for medical tubing. In this embodiment, the cam gripping mechanism 810 is placed within the fastener in the two cavities in the fastener halves. As with the other embodiments, the fastener includes a first piece that is a substantially planar piece having an exterior face, an interior face that is enclosed within the fastener when the fastener is closed, a rear side, and front side, and a left and right side.

The interior faces of the first piece and second pieces include an engaging channel 840 of sufficient width to retain the cam mechanism 810 within the fastener. The cam mechanism, as shown in Figures 41 - 43, has a first cam 801 with a series of gripping teeth 802 and a second cam 803 with a series of gripping teeth 804, both cams 801, 803 proximate the same side of the engaging channel 840 and a first cog 806 and a second cog 807, each with a series of gripping teeth 808 along the opposing side of the engaging channel 840 sufficient to retain an IV tubing between the first and second cam gripping teeth 802, 804 and the gear gripping teeth 808. The series of cam gripping teeth 802, 804 each are biased towards the engaging channel 809 by at least one arm 811, 812 extending from the opposing engaging channel wall and a rotary cam piece biased against the opposing engaging channel wall.

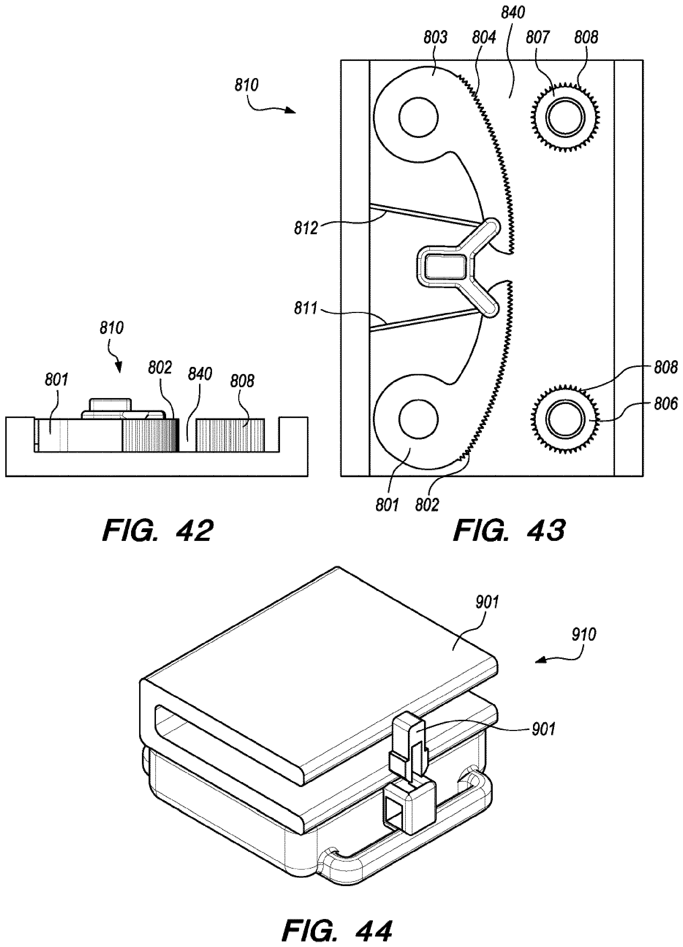

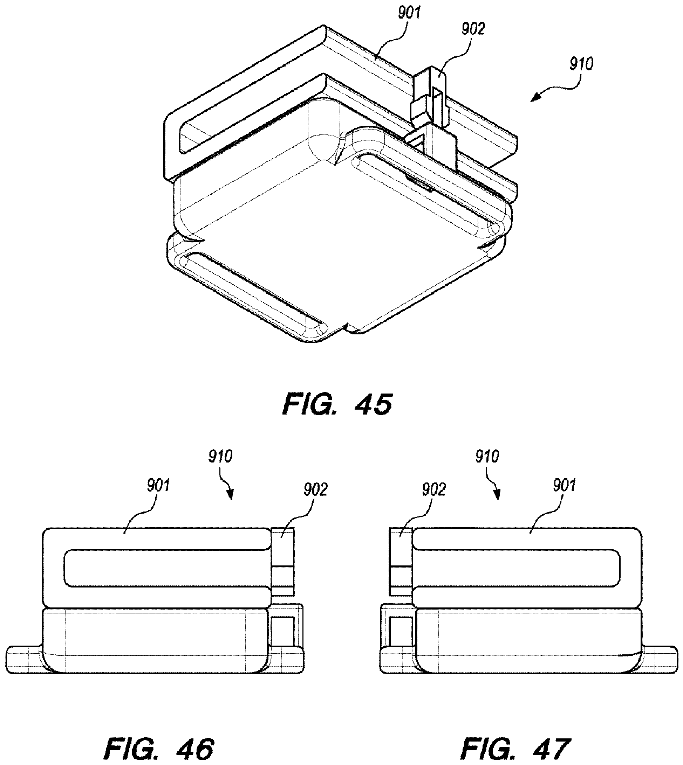

Fig. 44 - 47 show the tenth embodiment of the fastener 910 for medical tubing. As with the other embodiments, the fastener includes a first piece that is a substantially planar piece having an exterior face, an interior face that is enclosed within the fastener when the fastener is closed, a rear side, and front side, and a left and right side. As shown, the fastener 910 includes a flexible clip 901 that is configured to secure around the IV tube by buckling to the fastener 910 at one end of the clip via a buckle 902. In the preferred embodiment, the clip 901 is made of thin sheet plastic, gel, and high friction polymer. Other materials are suitable and are included within the scope of the embodiment. The flexible clip may use any of the variety of latching mechanisms that are known, e.g. the clip, the buckle on rigger belts, zip-tie straps and hook-and-loop cable tie straps. Preferentially, the flexible clip may incorporate high friction or adhesive materials to aid its gripping action. Alternatively, an integral adhesive band/tape may be wrapped around the IV tube to anchor it to the bracelet-plate.

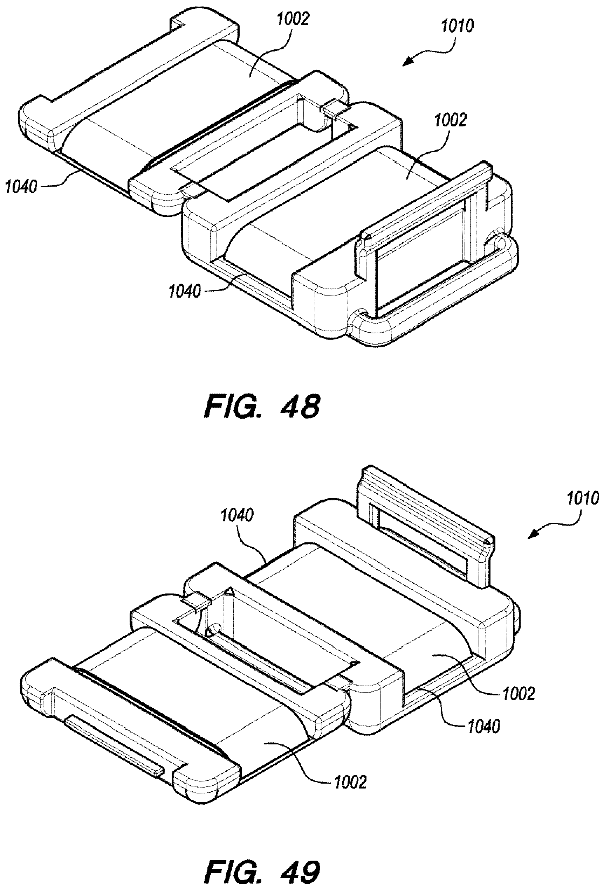

Fig. 48 - 51 show the eleventh embodiment of the fastener 1010 for medical tubing. Fig. 48 is a top perspective view showing the fastener 1010 for medical tubing having an arch spring 1002 in each of the engaging channels 1040. In this embodiment, the IV tube in the engaging channels 1040 is gripped by the arch springs 1002, which may include a layer of high friction polymer on the side contacting the IV tube or a layer of teeth or texture to contact the tubing. As with the other embodiments, the fastener 1010 includes a first piece 12 that is a substantially planar piece having an exterior face 18, an interior face 20 that is enclosed within the fastener 1010 when the fastener is closed, a rear side 22, and front side 24, and a left and right side 26, 28. A closure flap 30 having a locking projection 32 is secured to the front side 24 of the first piece 12 and is configured to connect and secure to a corresponding inlet 34 on the second piece 14 to secure the first piece to the second piece along the front face 24 of the fastener 1010.

The interior face 20 of the first piece 12 includes an engaging channel 1040 located in the center of the face and running parallel to the longitudinal axis of the fastener from the left 26 to the right side 28 of the fastener 1010. The engaging channel 1040 is of sufficient width to retain the arch spring clips 1002 placed within the fastener 1010.

The second piece 14 is a substantially planar piece having an exterior face 40, an interior face 42 that is enclosed within the fastener 1010 when the fastener is closed, a rear side 44, and front side 46, and a left and right side 48, 50. The closure flap 30 locking projection 32 of the first piece 12 is configured to connect and secure to the corresponding inlet 34 on the front side 46 of the second piece 14 to secure the first piece to the second piece along the front face 46 of the fastener 1010.

The interior face 42 of the second piece 14 includes an engaging channel 1040 located in the center of the face and running parallel to the longitudinal axis of the fastener from the left 48 to the right side 50 of the fastener 1010 piece 14. The engaging channel 1040 is configured to retain within the channel 52 the arch spring clip 1002.

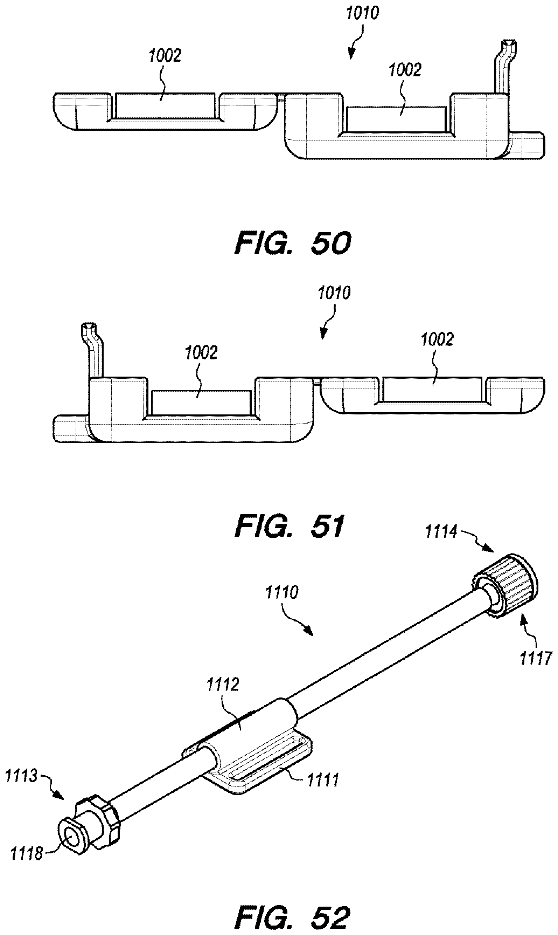

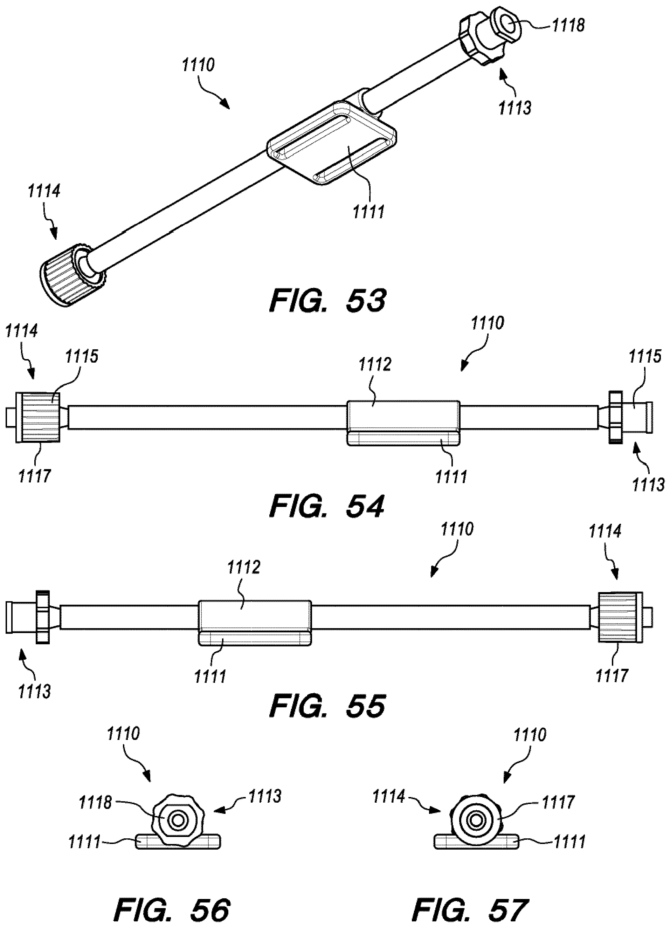

Fig. 52 - 57 show the twelfth embodiment of the fastener 1110 for medical tubing. Fig. 52 shows the buckle 1111 for securing to the patient having an IV extension 1110 secured to the buckle by a channel 1112. The buckle 1111 is configured to receive a strap to secure to the patient. The IV extension 1110 has two opposing ends for connection to an IV and extends a standard length, such as six to eight inches from one opposing end 1113 to the other opposing end 1114, with a tubular fluid pathway disposed within the length of the extension. Each opposing end has a connector 1115, one of which may be a female connecting end or a male connecting end. As shown, the connecting ends 1115 may or may not incorporate mechanisms that automatically stop the fluid flow when the IV line is disconnected from the extension.

The fastener 1110 connecting ends 1115 may include luer connectors having a shroud 1117 with a threaded connection 1119 or the connecting ends may include luer connectors having a valve formed from a resilient, elastomeric member 1118 within the central part of the luer connector. Information on elastomeric valves can be found in U.S. Patent No. 5676346 issued to Leinsing on October 17, 1997, which is hereby incorporated by reference in its entirety.

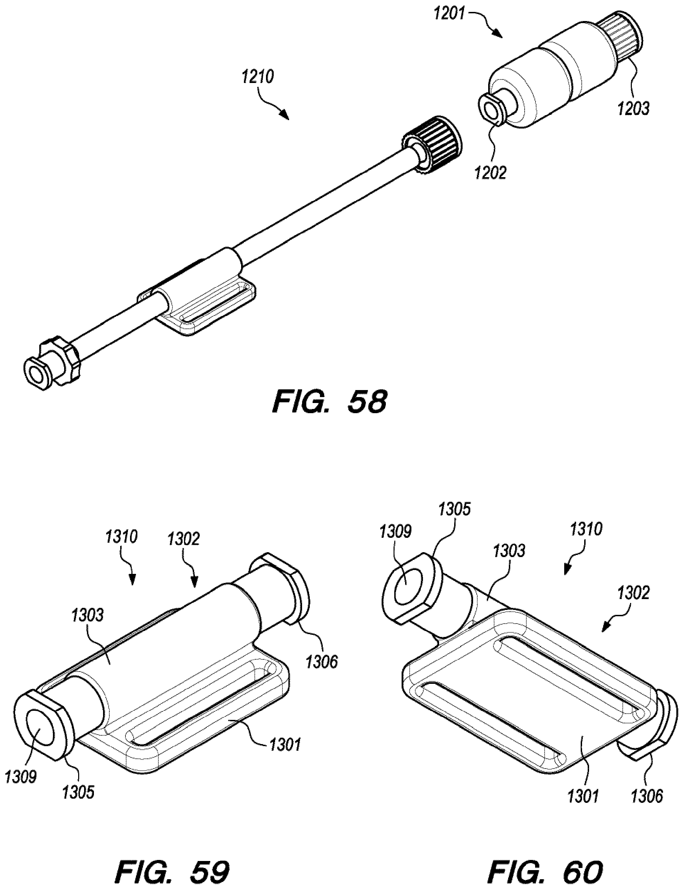

Fig. 58 shows the thirteenth embodiment of the fastener 1210 for medical tubing. In addition to the embodiment of the Figs. 48 - 57, Fig. 58 shows a breakaway device 1201 for connection between the extension fastener 1110 and the IV. In this manner, if the fastener is connected to the patient and the breakaway device 1201 is connected to the IV and the patient moves suddenly such that the IV would be dislodged, this invention would allow for the fastener to move and the IV breakaway to stay with the patient. The breakaway device 1201 may include opposing luer connector ends that have a shroud 1202 with a threaded connection or the connecting ends may include luer connectors having a valve formed from a resilient, elastomeric member 1202 within the central part of the luer connector. A tubular fluid pathway is disposed within the length of the breakaway device 1201.

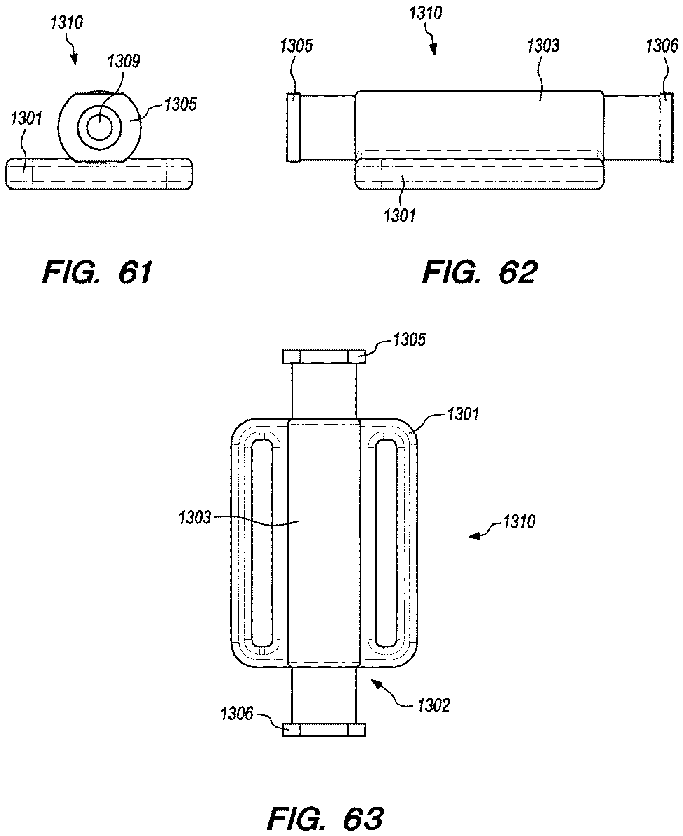

Fig. 59 - 63 show the fourteenth embodiment of the fastener 1310 for medical tubing. Fig. 59 shows the buckle 1301 for securing to the patient having a double luer connection 1302 secured to the buckle by a channel 1303. The buckle 1301 is configured to receive a strap to secure to the patient. The double luer connection 1302 has two opposing ends for connection to an IV and extends a standard length from one opposing end 1305 to the other opposing end 1306, with a tubular fluid pathway disposed within the length of the extension. Each opposing end has a connector, one of which may be a female connecting end or a male connecting end. As shown, the connecting ends may or may not incorporate mechanisms that automatically stop the fluid flow when the IV line is disconnected from the extension.

The double luer connection 1302 connecting ends include luer connectors having a valve formed from a resilient, elastomeric member 1309 within the central part of the luer connector.

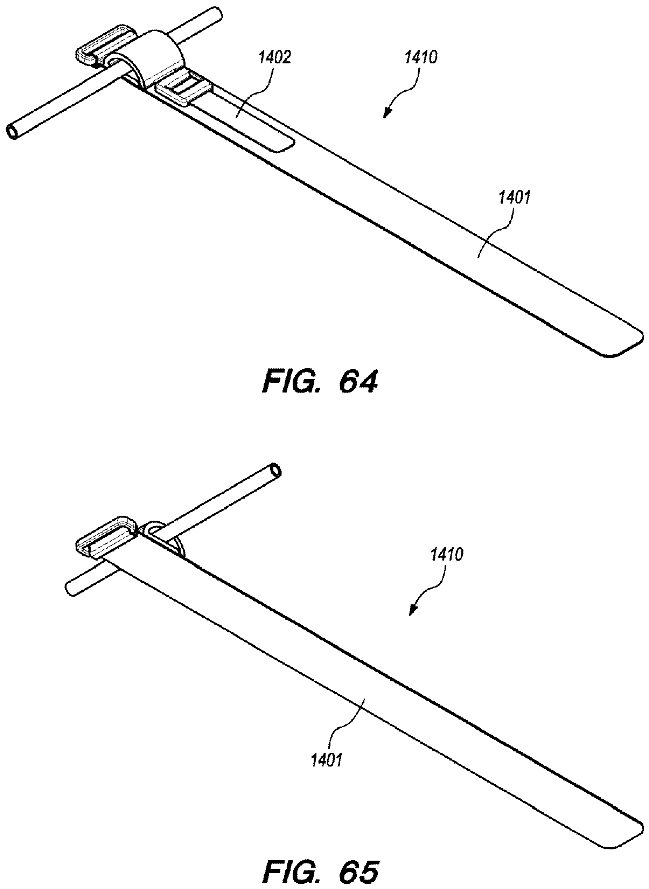

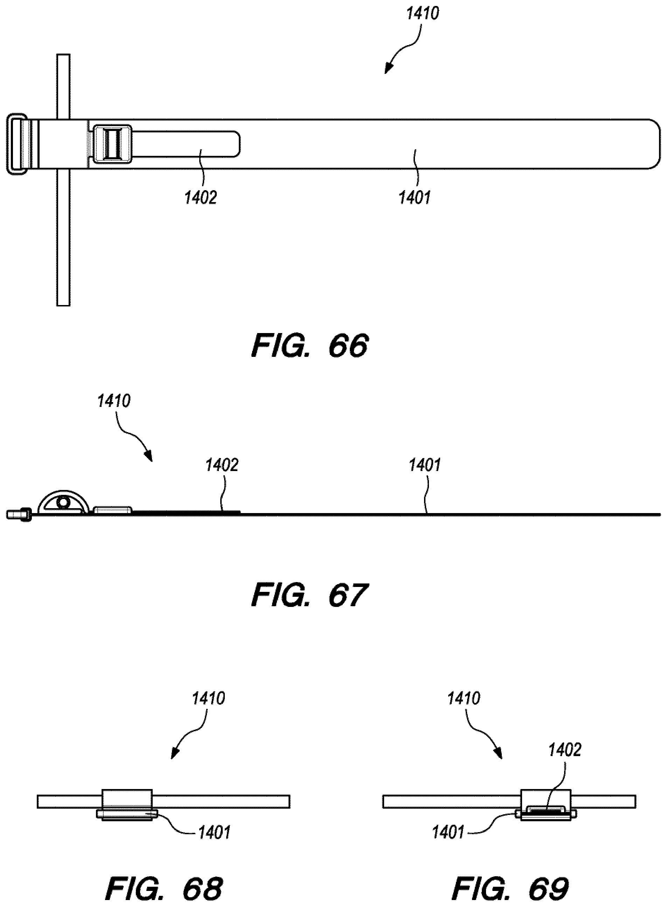

Fig. 64 - 69 show the fifteenth embodiment of the fastener 1410 for medical tubing. Fig. 64 shows the flexible fastener 1410 having a flexible strap 1401 for securing to the patient's body, that is cinched around the IV tubing to grip tubes of various diameters. The cinching mechanisms 1402 may be any of those that are known, e.g. the buckle on rigger belts, zip-tie straps and hook-and-loop cable tie straps. Preferentially, the tube-gripping strap may incorporate high friction or adhesive materials to aid its gripping action. Alternatively, an integral adhesive band/tape may be wrapped around the IV tube to anchor it to the bracelet-plate.

All terms used herein should be interpreted in the broadest possible manner consistent with the context. In particular, the terms "comprises" and "comprising" should be interpreted as referring to elements, components, or steps in a non-exclusive manner, indicating that the referenced elements, components, or steps may be present, or utilized, or combined with other elements, components, or steps that are not expressly referenced. When a Markush group or other grouping is used herein, all individual members of the group and all combinations and subcombinations possible of the group are intended to be individually included. All references cited herein are hereby incorporated by reference to the extent that there is no inconsistency with the disclosure of this specification. When a range is stated herein, the range is intended to include all sub-ranges within the range, as well as all individual points within the range. When "about," "approximately," or like terms are used herein, they are intended to include amounts, measurements, or the like that do not depart significantly from the expressly stated amount, measurement, or the like, such that the stated purpose of the apparatus or process is not lost.

The present invention has been described with reference to certain preferred and alternative embodiments that are intended to be exemplary only and not limiting to the full scope of the present invention, as set forth in the appended claims.

## Claims

1. A fastener for securing medical tubing to a patient, the fastener comprising:
a housing having a first piece having an interior surface and a second piece having an opposing interior surface, the housing extending a length along a longitudinal axis;
an engaging channel extending a length along the longitudinal axis of the housing on the interior surface of the second piece from the first side of the second piece to the opposing second side of the second piece, the engaging channel having a first channel side and an opposing second channel side each extending the length of the engaging channel, the engaging channel further comprising a series of engaging projections configured to grip the medical tubing, each engaging projection of the series of engaging projections extending from the engaging channel from the first channel side of the engaging channel to the second channel side and distributed along the length of the engaging channel; and
an engaging strip extending from the interior surface of the first piece along at least a portion of the longitudinal length of the housing.

2. The fastener of claim 1, further comprising a series of supporting ribs extending from the interior surface of the first piece along the longitudinal axis of the first piece.

3. The fastener of claim 1 or 2, wherein the engaging strip comprises a multitude of teeth distributed along the engaging strip.

4. The fastener of claim 1, 2 or 3, wherein the engaging strip further comprises a coating selected from the group consisting of a high friction polymer and an adhesive.

5. The fastener of any of claims 1 to 4, further comprising a closure configured to secure the first piece to the second piece.

6. The fastener of any preceding claim, wherein the housing includes a
a lid extending a length along the longitudinal axis, the lid having an arcuate exterior and an interior surface and a base in a hingeable relationship with the lid, the base extending a length along a longitudinal axis from a first side to an opposing second side and having an interior surface, the engaging channel being arcuate and extending a length along the longitudinal axis of the base from the first side of the base to the opposing second side of the base.

7. The fastener of claim 6, wherein the engaging strip extends from the interior surface of the lid along the entire longitudinal length of the lid.
